# EUROPEAN PATENT SPECIFICATION

(11) **EP 0 812 194 B1**
(45) Date of publication and mention of the grant of the patent: **16.08.2000**
(21) Application number: 96908641.2
(22) Date of filing: 29.02.1996
(51) Int. Cl.: A61K 31/44

(54) **Use of substituted pyridine compounds for treating atopic dermatitis and contact dermatitis**
Verwendung von substituirten Pyridinverbindungen zur Behandlung der atopischen Dermatitis und der Kontaktdermatitis
Utilisation de composés substitués de pyridine pour le traitement de l'eczema constitutionel et de l'eczema de contact

(30) Priority: 02.03.1995 US 397461
(43) Date of publication of application: 17.12.1997
(73) Proprietor: SMITHKLINE BEECHAM CORPORATION, King of Prussia, PA 19406-0939 (US)
(72) Inventor: GRISWORLD, Don, Edgar, North Wales, PA 19454 (US)
(74) Representative: Waters, David Martin, Dr.
(86) International application number: US9602950
(87) International publication number: WO9626725

(56) References cited:
- WO-A-93/06085
- WO-A-94/00437
- RUZICKA T ET AL: "LEUKOTRIENES IN SKIN OF ATOPIC DERMATITIS" JOURNAL OF INVESTIGATIVE DERMATOLOGY, vol. 82, no. 5, May 1984, page 563 XP000608907
- S. THORSEN ET AL: "Leukotriene B4 in atopic dermatitis: increased skin levels and altered sensitivity of peripheral blood T-cells" ALLERGY, vol. 45, 1990, pages 457-463, XP002050582
- D. J. FRETLAND ET AL: "Antiinflammatory Effects of Second-Generation Leukotriene B4 Receptor Antagonist, SC-53228: Impact upon Leukotriene B4- and 12(R)-HETE-Mediated Events" INFLAMMATION, vol. 19, no. 2, 1995, pages 193-205, XP002050583
- DJURIC S W ET AL: "THE LEUKOTRIENE B4 RECEPTOR ANTAGONISTS - A MOST DISCRIMINATING CLASS OF ANTIINFLAMMATORY AGENT?" DRUGS OF THE FUTURE, vol. 17, no. 9, 1992, pages 819-830, XP000650327
- CHEMICAL ABSTRACTS, CA 113:217815, YAMASHITA T., "Skin-Lightening Preparations Containing Fusaric Acid and/or Picolinic Acids"; & JP,A,02 164 808, 25 June 1990.

## Description

### Scope of the Invention

This invention relates to the use of certain compounds containing a substituted pyridyl group linked to a substituted phenyl group by an alkyl or heteroatom-containing tether for treating atopic dermatitis or contact dermatitis in mammal.

### Background of the Invention

The family of bioactive lipids known as the eicosanoids exert pharmacological effects on cutaneous, respiratory, cardiovascular, and gastrointestinal systems. The leukotrienes are generally divided into two subclasses, the peptidoleukotrienes (leukotrienes C₄, D₄ and E₄) and the dihydroxyleukotrienes (leukotriene B₄). This invention is primarily concerned with the hydroxyleukotrienes (LTB₄) but is not limited to this specific group of leukotrienes. In fact, the receptor with which LTB₄ interacts appears to also be utilized by a variety of other eicosanoids, including 12-HETE and dihydro 12-HETE. These receptors are either the same or closely-related so that compounds of this invention inhibit the action of several eicosanoids by antagonising this receptor.

These eicosanoids are derived from arachidonic acid and are critically involved in mediating many types of cardiovascular, pulmonary, dermatological, renal, allergic, and inflammatory diseases including asthma, adult respiratory distress syndrome, cystic fibrosis, psoriasis, and inflammatory bowel disease.

By antagonizing the effects of eicosanoids that interact at the LTB₄ receptor, or other pharmacologically active mediators at the end organ, for example airway smooth muscle, the compounds and pharmaceutical compositions of the present invention are valuable in the treatment of diseases in subjects, including human or animals, in which these eicosanoids are a factor.

S Thorsen *et al, Allergy,* 1990, **45**, 457-463 and T. Ruzicka *et al, Journal of Investigative Dermatology,* May 1984, **82**(5), 563 disclose that elevated levels of LTB₄ are present in lesional skin in atopic dermatitis.

### Summary of the Invention

This invention relates the use of at least one compound of formula I 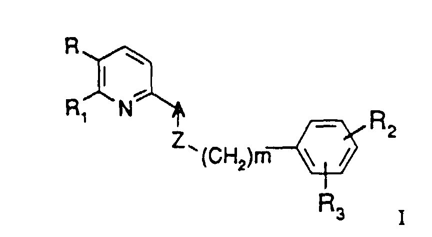 or an N-oxide, or a pharmaceutically acceptable salt thereof,
where
A is CH₂ and Z is S(O)_{q} when q is 0, 1 or 2; CHOH; C=O; NRₓ; or O; or
A is C=O and Z is NRₓ;
m is 0 - 5;
R is C₁ to C₂₀-aliphatic, unsubstituted or substituted phenyl C₁ to C₁₀-aliphatic where substituted phenyl has one or more radicals selected from the group consisting of lower alkoxy, lower alkyl, trihalomethyl, and halo, or R is C₁ to C₂₀-aliphatic-O-, or R is unsubstituted or substituted phenyl C₁ to C₁₀-aliphatic-O-where substituted phenyl has one or more radicals selected from the group consisting of lower alkoxy, lower alkyl, trihalomethyl, and halo;
R₁ is -(C₁ to C₅ aliphatic)R₄, -(C₁ to C₅ aliphatic)CHO, -(C₁ to C₅ aliphatic)CH₂OR₈, -R₄, -CH₂OH, or CHO;
R₂ is H, halo, lower alkyl, lower alkoxy, -CN, -(CH₂)ₙR₄, -CH(NH₂)(R₄), or -(CH₂)ₙR₉ where n is 0 - 5 and where R₉ is -N(R₇)₂ where each R₇ is independently H, or an aliphatic group of 1 to 10 carbon atoms, or acyl of 1-6 carbon atoms, or a cycloalkyl-(CH₂)ₙ- group of 4 to 10 carbons where n is 0-3, or both R₇ groups form a ring which includes the nitrogen and having 4 to 6 carbons; or
R₃ is hydrogen, lower alkyl, lower alkoxy, halo, -CN, R₄, NHCONH₂, or OH;
each R₄ group is independently -COR₅ where R₅ is -OH, a pharmaceutically acceptable ester-forming group -OR₆, or -OX where X is a pharmaceutically acceptable cation, or R₅ is -N(R₇)₂ where each R₇ is independently H, or an aliphatic group of 1 to 10 carbon atoms, or a cycloalkyl-(CH₂)ₙ- group of 4 to 10 carbons where n is 0-3, or both R₇ groups form a ring having 4 to 6 carbons, or R₄ is a sulfonamide, or an amide, or tetrazol-5-yl; and
R₈ is hydrogen, C₁ to C₆ alkyl, or C₁ to C₆-acyl;
in the manufacture of a medicament for use in treating atopic dermatitis.

### Detailed Description of the Invention

The following definitions are used in describing this invention and setting out what the inventors believe to be their invention herein.

"Aliphatic" is intended to include saturated and unsaturated radicals. This includes normal and branched chains, saturated or mono or poly unsaturated chains where both double and triple bonds may be present in any combination. The phrase "lower alkyl" means an alkyl group of 1 to 6 carbon atoms in any isomeric form, but particularly the normal or linear form. "Lower alkoxy" means the group lower alkyl-O-. "Halo" means fluoro, chloro, bromo or iodo. "Acyl" means the radical having a terminal carbonyl carbon.

When reference is made to a substituted phenyl ring, it is meant that the ring can be substituted with one or more of the named substituents as may be compatible with chemical synthesis. Multiple substituents may be the same or different, such as where there are three chloro groups, or a combination of chloro and alkyl groups and further where this latter combination may have different alkyl radicals in the chloro/alkyl substituent pattern.

The phrase "a pharmaceutically acceptable ester-forming group" in R₂ and R₃ covers all esters which can be made from the acid function(s) which may be present in these compounds. The resultant esters will be ones which are acceptable in its application to a pharmaceutical use. By that it is meant that the mono or diesters will retain the biological activity of the parent compound and will not have an untoward or deleterious effect in their application and use in treating diseases. Such esters are, for example, those formed with one of the following radicals: C₁ to C₆ alkyl, phenyl C₁-C₆alkyl, cycloalkyl, aryl, arylalkyl, alkylaryl, alkylarylalkyl, aminoalkyl, indanyl, pivaloyloxymethyl, acetoxymethyl, propionyloxymethyl, glycyloxymethyl, phenylglycyloxymethyl, or thienylglycyloxymethyl. The most preferred ester-forming radicals are those where R₃ is alkyl, particularly alkyl of 1 to 10 carbons, (ie CH₃-(CH₂)ₙ- where n is 0-9), or phenyl-(CH₂)ₙ- where n is 0-4.

When R₂ is referred to as being an amine, that includes the radical -NH₂ and mono- or dialkylate derivatives of this -NH₂ radical. Preferred alkylated amines are the mono- or disubstituted amines having 1 to 6 carbons. When R₂ is referred to as being an amide, that includes all acylate derivatives of the NH₂ radical. The preferred amides are those having 1 to 6 carbons.

Where there is an acid group, amides may be formed. The most preferred amides are those where -R₆ is hydrogen or alkyl of 1 to 6 carbon atoms. Particularly preferred is the diethylamide or dimethylamide.

Pharmaceutically acceptable salts of the instant compounds are intended to be covered by this invention. These salts will be ones which are acceptable in their application to a pharmaceutical use. By that it is meant that the salt will retain the biological activity of the parent compound and the salt will not have untoward or deleterious effects in its application and use in treating diseases.

Pharmaceutically acceptable salts are prepared in a standard manner, in a suitable solvent. The parent compound in a suitable solvent is reacted with an excess of an organic or inorganic acid, in the case of acid addition salts, or an excess of organic or inorganic base in the case where R₄ is OH.

N-oxides may also be prepared by means of selected oxidizing agents. These oxides are useful as intermediates in preparing the compounds of formula I and have useful pharmaceutical activity in and of themselves. Hence one can administer the N-oxides of formula I to a subject who is susceptible to or is suffering from a disease related to or caused by LTB₄ or similar eicosanoids which react at that receptor or similar receptor.

If by some combination of substituents, a chiral center is created or another form of an isomeric center is created in a compound of this invention, all forms of such isomer(s) are intended to be covered herein. These compounds may be used as a racemic mixture or the racemates may be separated and the individual enantiomer used alone. Olefins may have the cis or trans configuration (E or Z); either are useful in the practice of this invention.

The preferred compounds are those where Z is O or S(O)_{q}; m is 0-3; n is 0-2; R is alkoxy of 8 to 15 carbon atoms or unsubstituted or substituted phenyl-C₁ to C₁₀-aliphatic-O-; and R₁ is -(C₁ to C₅ aliphatic)R₄ or -(C₁ to C₅-aliphafic)CH₂OR₈. The more preferred compounds of this invention are those where R₁ is R₄CH=CH- and R₂ is -COR₅ or -NHSO₂CF₃. Another set of preferred compounds are the anilines, those where R₂ is N(R₇)₂, particularly where R₇ is hydrogen. A third set of preferred compounds are those where both R₂ and R₃ are hydrogen. Yet another set of preferred compounds are the 2,6-dihalophenyl analogs, particularly the 2,6-dichlorophenyl compounds.

The illustrated compounds

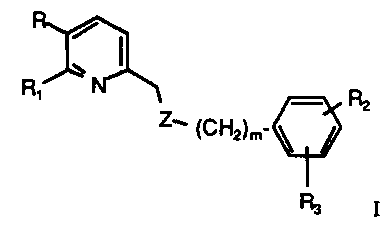

| Z-(CH₂)ₘ- | R | R₁ | R₂ |
|---|---|---|---|
| S (m is 0) | H₂₅C₁₂-O- | *HOOC-CH=CH- | *m*-COOH |
| S (m is 0) | H₂₅C₁₂-O- | *HOOC-CH=CH- | *p*-COOH |
| S (m is 0) | H₂₅C₁₂-O- | *HOOC-CH=CH- | *o*-COOH |
| S=O (m is 0) | H₂₅C₁₂-O- | *HOOC-CH=CH- | *m*-COOH |
| S=O (m is 0) | H₂₅C₁₂-O- | *HOOC-CH=CH- | *p*-COOH |
| S=O (m is 0) | H₂₅C₁₂-O- | *HOOC-CH=CH- | *o*-COOH |
| SO₂ (m is 0) | H₂₅C₁₂-O- | *HOOC-CH=CH- | *m*-COOH |
| O (m is 0) | H₂₅C₁₂-O- | *HOOC-CH=CH- | *m*-COOH |
| O (m is 0) [N-oxide] | CH₃O-Ph-(CH₂)₈-O- | *HOOC-CH=CH- | *m*-COOH |
| O (m is 0) [N-oxide] | H₂₅C₁₂-O- | *HOOC-CH=CH- | *m*-COOH |

| | | | |
|---|---|---|---|
| ** Trans configuration.* | | | |

In addition, the following named compounds are illustrated herein:
1-fluoro-3-[2-thia-3-[2-(E-2-carboxyethenyl)-3-[4-(4-methoxyphenyl)butyloxy]-6-pyridyl]propyl]benzene, lithium salt;
3-[2-aria-3-[2-(E-2-carboxyethenyl)-3-[4-(4-methoxyphenyl)butyloxy]-6-pyridyl]propyl]benzene, lithium salt;
3-[2-thia-3-[2-(2-carboxyethanyl)-3-[4-(4-methoxyphenyl)butyloxy]-6-pyridyl]propyl]benzene, lithium salt;
2-[2-thia-3-[2-(2-carboxyethenyl)-3-[4-(4-methoxyphenyl)butyloxy]-6-pyridyl]ethyl]benzene, lithium salt;
1-fluoro-4-[2-thia-3-[2-(2-carboxyethanyl)-3-[4-(4-methoxyphenyl)butyloxy]-6-pyridyl]propyl]benzene, lithium salt;
1-fluoro-4-[2-thia-3-[2-(E-2-carboxyethenyl)-3-[4-(4-methoxyphenyl)butyloxy]-6-pyridyl]propyl]benzene, lithium salt;
3-[1-this-2-[2-(E-2-carboxyethenyl)-3-[8-(4-methoxyphenyl)octyloxy]-6-pyridyl]ethyl]benzoic acid,
3-[1-oxythia-2-[2-(E-2-cuboxyethenyl)-3-[8-(4-methoxyphenyl)octyloxy]-6-pyridyl]ethyl]benzoic acid,
3-[1-thia-2-[2-(E-2-carboxyethenyl)-3-dodecyloxy-6-pyridyl]ethyl]benzoic acid,
3-[1-oxythia-2-[2-(E-2-carboxyethenyl)-3-dodecyloxy-6-pyridyl]ethyl]benzoic acid,
3-[1-dioxythia-2-[2-(E-2-carboxyethenyl)-3-dodecyloxy-6-pyridyl]ethyl]benzoic acid,
2-[1-oxythia-2-[2-(E-2-carboxyethenyl)-3-dodecyloxy-6-pyridyl]ethyl]benzoic acid, lithium salt
N-[3-[1-thia-2-[2-(E-2-carboxyethenyl)-3-dodecyloxy-6-pyridyl]ethyl]phenyl]trifluoromethanesulfonamide,
N-[3-[1-thia-2-[2-(E-2-carboxyethenyl)-3-(8-(4-methoxyphenyl)octyloxy)-6-pyridyl]ethyl]phenyl]trifluoromethanesulfonamide,
N-[3-[1-oxythia-2-[2-(E-2-carboxyethenyl)-3-(8-(4-methoxyphenyl)octyloxy)-6-pyridyl]ethyl]phenyl]-trifluoromethanesulfonamide,
N-[3-[1-oxythia-2-[2-(E-2-carboxyethenyl)-3-(8-(4-methoxyphenyl)octyloxy)-6-pyridyl]ethyl]phenyl]-phenylsulfonamide,
N-[3-[1-thia-2-[2-(E-2-carboxyethenyl)-3-(8-(4-methoxyphenyl)octyloxy)-6-pyridyl]ethyl]-phenyl]phenylsulfonamide,
3-[1-oxa-2-[2-(E-2-carboxyethenyl)-3-dodecyloxy-6-pyridyl]ethyl]benzoic acid,
3-[1-oxa-2-[2-(E-2-carboxyethenyl)-3-[8-(4-methoxyphenyl)octyloxy]-6-pyridyl]ethyl]benzoic acid,
3-[1-oxa-2-[2-(E-2-carboxyethenyl)-3-[8-(4-methoxyphenyl)octan-1-yl]-6-pyridyl]ethyl]benzoic acid,
4-[2-thia-3-[2-(E-2-carboxyethenyl)-3-[8-(4-methoxyphenyl)octyloxy]-6-pyridyl]propyl]benzoic acid,
4-[2-thia-3-[2-(E-2-carboxyethenyl)-3-[4-(4-methoxyphenyl)butyloxy]-6-pyridyl]propyl]benzoic acid,
3-[2-thia-3-[2-(E-2-carboxyethenyl)-3-[8-(4-methoxyphenyl)octyloxy]-6-pyridyl]propyl]benzoic acid,
3-[2-thia-3 [2-(E-2-carboxyethenyl)-3-[4-(4-methoxyphenyl)butyloxy]-6-pyridyl]propyl]benzoic acid,
3-[2-thia-3-[2-(2-carboxyethanyl)-3-[8-(4-methoxyphenyl)octyloxy]-6-pyridyl]propyl]benzoic acid,
3-[2-thia-3-[2-(E-2-carboxyethenyl)-3-(8-(4-methoxyphenyl)octyloxy)-6-pyridyl]propyl]-N,N,-dimethylbenzamide, lithium salt
3-[2-thia-3-[2-(E-2-carboxyethenyl)-3-(4-(4-methoxyphenyl)butyloxy)-6-pyridyl]propyl]-N,N-dimethylbenzamide, lithium salt,
3-[2-thia-3-[2-(E-2-carboxyethenyl)-3-[4-phenylbutyloxy]-6-pyridyl]propyl]benzoic acid,
3-[2-thia-3-[2-(E-2-carboxyethenyl)-3-[8-phenyloctyloxy]-6-pyridyl]propyl]benzoic acid,
3-[2-thia-3-[2-(2-carboxyethanyl)-3-[4-(4-methoxyphenyl)butyloxy]-6-pyridyl]propyl]benzoic acid,
4-[2-thia-3-[2-(E-2-carboxyethenyl)-3-[8-(4-methoxyphenyl)octyloxy]-6-pyridyl]propyl]phenylacetic acid,
4-[2-oxythia-3-[2-(E-2-carboxyethenyl)-3-[8-(4-methoxyphenyl)octyloxy]-6-pyridyl]propyl]benzoic acid,
3-[2-oxythia-3-[2-(E-2-carboxyethenyl)-3-[8-(4-methoxyphenyl)octyloxy]-6-pyridyl]propyl]benzoic acid,
4-[2-oxythia-3-[2-(E-2-carboxyethenyl)-3-[8-(4-methoxyphenyl)octyloxy]-6-pyridyl]propyl]phenylacetic acid,
3-[2-dioxythia-3-[2-(E-2-carboxyethenyl)-3-[8-(4-methoxyphenyl)octyloxy]-6-pyridyl]propyl]benzoic acid,
5-[3-[2-thia-3 [2-(E-2-carboxyethenyl)-3-[8-(4-methoxyphenyl)octyloxy]-6-pyridyl]propyl]phenyl]tetrazole
3-[1-oxa-2-[2-(E-2-carboxyethenyl)-3-(8-(4-methoxyphenyl)octyloxy)-6-pyridyl]ethyl]aniline,
5-carboxy-3-[1-oxa-2-[2-(E-2-carboxyethenyl)-3-(8-(4-methoxyphenyl)octyloxy)-6-pyridyl]ethyl]aniline,
3-[1-thia-2-[2-(E-2-carboxyethenyl)-3-(8-(4-methoxyphenyl)octyloxy)-6-pyridyl]ethyl]aniline,
3-[1-thia-2-[2-(E-2-carboxyethenyl)-3-(8-(4-trifluoromethylphenyl)octyloxy)-6-pyridyl]ethyl]aniline,
3-[1-oxythia-2-[2-(E-2-carboxyethenyl)-3-(8-(4-trifluoromethylphenyl)octyloxy)-6-pyridyl]ethyl]aniline, lithium salt
3-[1-thia-2-[2-(E-2-carboxyethenyl)-3-(8-phenyloctyloxy)-6-pyridyl]ethyl]aniline, lithium salt
3-[1-oxythia-2-[2-(E-2-carboxyethenyl)-3-(8-(4-fluorophenyl)octyloxy)-6-pyridyl]ethyl]aniline,
3-[1-oxythia-2-[2-(E-2-carboxyethenyl)-3-(8-phenyl)octyloxy)-6-pyridyl]ethyl]aniline,
3-[1-thia-2-[2-(E-2-carboxyethenyl)-3-(8-(4-methoxyphenyl)octyloxy)-6-pyridyl]ethyl]-N,N dimethylaniline,
3-[1-thia-2-[2-(E-2-carboxyethenyl)-3-(4-(4-methoxyphenyl)butyloxy)-6-pyridyl]ethyl]aniline, lithium salt
3-[1-oxythia-2-[2-(E-2-carboxyethenyl)-3-(4-(4-methoxyphenyl)butyloxy)-6-pyridyl]ethyl)aniline, lithium salt
3-[1-dioxythia-2-[2-(E-2-carboxyethenyl)-3-(4-(4-methoxyphenyl)butyloxy)-6-pyridyl]ethyl]aniline, lithium salt
3-[2-thia-3-[2-(E-2-carboxyethenyl)-3-(8-(4-methoxyphenyl)octyloxy)-6-pyridyl]propyl]-N,N-dimethylaniline,
3-[1-oxythia-2-[2-(E-2-carboxyethenyl)-3-(8-(4-methoxyphenyl)octyloxy)-6-pyridyl]ethyl)aniline,
3-[1-oxythia-2-[2-(E-2-carboxyethenyl)-3-(8-(4-methoxyphenyl)octyloxy)-6-pyridyl]ethyl]-N,N-dimethylaniline,
3-[1-dioxythia-2-[2-(E-2-carboxyethenyl)-3-(8-(4-methoxyphenyl)octyloxy)-6-pyridyl]ethyl]aniline,
(E)-lithium 3-[3-[4-(4-methoxyphenyl)butyloxy]-6-[(2-phenylthio)methyl]-2-pyridinyl]-2-propenoate,
(E)-lithium 3-[3-[4-(4-methoxyphenyl)butyloxy]-6-[(3,4-dichlorophenylthio)methyl]-2-pyridinyl]-2-propenoate,
(E)-lithium 3-[3-[4-(4-methoxyphenyl)butyloxy]-6-[(4-chlorophenylthio)methyl]-2-pyridinyl]-2-propenoate,
(E)-sodium 3-[3-[4-(4-methoxyphenyl)butyloxy]-6-[(4-fluorophenylthio)methyl]-2-pyridinyl]-2-propenoate,
(E) lithium 3-[3-[4-(4-methoxyphenyl)butyloxy]-6-[(2-chlorophenylthio)methyl]-2-pyridinyl]-2-propenoate,
(E)-sodium 3-[3-[4-(4-methoxyphenyl)butyloxy]-6-[(2-chlorobenzylthio)methyl]-2-pyridinyl]-2-propenoate,
(E)-sodium 3-[3-[4-(4-methoxyphenyl)butyloxy)-6-[(2-methylphenylthio)methyl]-2-pyridinyl]-2-propenoate,
(E)-sodium 3-[3-[4-(4-methoxyphenyl)butyloxy]-6-[(3-chlorophenylthio)methyl]-2-pyridinyl]-2-propenoate,
(E)-sodium 3-[3-[4-(4-methoxyphenyl)buryloxy]-6-[(2-methoxyphenylthio)methyl]-2-pyridinyl]-2-propenoate,
(E)-sodium 3-[3-[4-(4-methoxyphenyl)butyloxy]-6[(2,4-dichlorophenylthio)methyl]-2-pyridinyl]-2-propenoate,
(E)-sodium 3-[3-[4-(4-methoxyphenyl)butyloxy]-6-[(2-bromophcnylthio)methyl]-2-pyridinyl]-2-propenoate,
(E)-sodium 3-[3-[4-(4-methoxyphenyl)butyloxy]-6-[(2-methylphenylthio)methyl]-2-pyridinyl]-2-propenoate,
(E)-sodium 3-[3-[4-(4-methoxyphenyl)butyloxy]-6[(2,6-fluorophenylthio)methyl]-2-pyridinyl]-2-propenoate,
(E)-sodium 3-[3-[4-(4-methoxyphenyl)butyloxy]-6-[(2,6-dimethylphenylthio)methyl]-2-pyridinyl]-2-propenoate,
(E)-sodium 3-[3-[4-(4-methoxyphenyl)butyloxy]-6-[(2,6-dimethoxyphenylthio)methyl]-2-pyridinyl]-2-propenoate,
(E)-lithium 3-[3-[4-(4-methoxyphenyl)butyloxy]-6-[(2,6-dichlorophenylthio)methyl]-2-pyridinyl]-2-propenoate,
(E)-sodium 3-[3-[4-(4-fluorophenyl)butyloxy]-6-[(2,6-dichlorophenylthio)methyl]-2-pyridinyl]-2-propenoate,
(E)-sodium 3-[3-[4-(4-fluorophenyl)butyloxy]-6-[(2,6-difluorophenylthio)methyl]-2-pyridinyl]-2-propenoate,
(E)-sodium 3-[3-[2-(4-fluorophenyl)ethyloxy]-6-[(2,6-dichlorophenylthio)methyl]-2-pyridinyl]-2-propenoate,
(E)-sodium 3-[3-[2-(4-fluorophenyl)ethyloxy]-6-[(2,6-difluorophenylthio)methyl]-2-pyridinyl]-2-propenoate,
(E)-sodium 3-[3-(4-fluorobenzyloxy)-6-[(2,6-dichlorophenylthio)methyl]-2-pyridinyl]-2-propenoate,
(E)-sodium 3-[3-[4-phenylbutyloxy]-6-[(2,6-dichlorophenylthio)methyl]-2-pyridinyl]-2-propenoate,
(E)-sodium 3-[3-[2-phenylethyloxy]-6-[(2,6-dichlorophenylthio)methyl]-2-pyridinyl]-2-propenoate,
(E)-sodium 3-[3-[2-(4-fluorophenyl)ethyloxy]-6-[(2,6-dichlorophenylthio)methyl]-2-pyridinyl]-2-propanoate,
(E)-sodium 3-[3-[4-(4-fluorophenyl)butyloxy]-6-[(2,6-dichlorophenylthio)methyl]-2-pyridinyl]-2-propanoate,
(E)-sodium 3-[3-[4-(4-methoxyphenyl)butyloxy]-6-[(2,6-dichlorophenylthio)methyl]-2-pyridinyl]-2-propanoate,
(E)-sodium 3-[3-[2-(4-methoxyphenyl)ethyloxy]-6-[(2,6-dichlorophenylthio)methyl]-2-pyridinyl]-2-propenoate,
(E)-sodium 3-[3-[2-(4-fluorophenyl)ethyloxy]-6-[(2,4,6-trichlorophenylthio)methyl]-2-pyridinyl]-2-propenoate,
(E)-sodium 3-[3-[2-(4-fluorophenyl)ethyloxy]-6-[(2-chloro-6-methylphenylthio)methyl]-2-pyridinyl]-2-propenoate,
(E)-sodium 3-[3-[2-(4-methoxyphenyl)ethyloxy]-6-[(2,6-dichlorophenylthio)methyl]-2-pyridinyl]-2-propanoate,
(E)-3-[3-[2-(4-chlorophenyl)ethyloxy]-6-[(2,6-dichlorophenylthio)methyl]-2-pyridinyl]-2-propenoic acid,
(E)-3-[3-[8-(4-methoxyphenyl)octyloxy]-6-[(2,6-dichlorophenylthio)methyl]-2-pyridinyl]-2-propenoic acid,
(E)-3-[3-[2-phenethyloxy]-6-[(2,6-dichlorophenylthio)methyl]-2-pyridinyl]-2-propenoic acid,
(E)-3-[3-[3-phenylpropyloxy]-6-[(2,6-dichlorophenylthio)methyl]-2-pyridinyl]-2-propenoic acid,
(E)-N,N-diethyl 3-[3-[2-phenethyloxy]-6-[(2,6-dichlorophenylthio)methyl)-2-pyridinyl]-2-propenamide,
(E)-3-[3-[2-phenethyloxy]-6-[(2,6-dichlorophenyloxy)methyl]-2-pyridinyl]-2-propenoic acid,
(E)-3-[3-[2-(thien-2-yl)ethyloxy]-6-[(2,6-dichlorophenylthio)methyl]-2-pyridinyl]-2-propenoic acid,
(E)-3-[3-[2-(thien-3-yl)ethyloxy]-6-[(2,6-dichlorophenylthio)methyl]-2-pyridinyl]-2-propenoic acid,
(E)-3-[3-[2-(3-methylthiazol-2-yl)ethyloxy]-6-[(2,6-dichlorophenylthio)methyl]-2-pyridinyl]-2-propenoic acid,
(E)-N,N-diethyl 3-[3-[2-phenethyloxy]-6-[(2,6-dichlorophenylthio)methyl)-2-pyridinyl]-2-propenamide,
(E)-3-[3-[2-phenethyloxy]-6-[(2,6-dichlorophenylsulfinyl)methyl)-2-pyridinyl]-2-propenoic acid,
(E)-3-[3-[2-phenethyloxy]-6-[(2,6-dichlorophenylsulfonyl)methyl]-2-pyridinyl]-2-propenoic acid,
3-[3-[2-phenethyloxy]-6-[(2,6-dichlorophenylthio)methyl]-2-pyridinyl]propanoic acid,
3-[3-[2-phenethyloxy]-6-[(2,6-dichlorophenylsulfinyl)methyl]-2-pyridinyl)propanoic acid,
3-[3-[2-phenethyloxy]-6-[(2,6-dichlorophenylsulfonyl)methyl]-2-pyridinyl]propanoic acid,
3-[3-[8-(4-methoxyphenyl)octyloxy]-6-[(2,6-dichlorophenylsulfinyl)methyl]-2-pyridinyl]propanoic acid,
3-[3-[8-(4-methoxyphenyl)octyloxy]-6-[(2,6-dichlorophenyldioxysulfonyl)methyl]-2-pyridinyl]propanoic acid, or
a free acid thereof or another pharmaceutically acceptable salt.

### Synthesis

The compounds of this invention can be made by the methods published in several patent applications, including but not limited to copending U.S. application 08/211063 filed 18 July 1994 and prior published as PCT/US92/07466 (WO93/06085) and copending U.S. application 08/163974 filed 08 December 1993 and prior published as PCT application serial number PCT/US93/06234 (WO94/00437).

As disclosed in the aforementioned publications there are several methods for preparing these compounds. One generic process comprises preparing a 6-(halomethyl)pyridyl adduct and then condensing this fragment with the appropriate mercaptan or alcohol to make compounds where Z is sulfur or oxygen. Usually, functional groups such as acid groups will be protected; any acid group may be derivatized in some manner to render it unreactive. After the condensation reaction, protecting groups may be removed to provide the parent functionality, e.g. an acid. Further modification of these reactive groups can then be carried out, such as forming a salt, an amide, an ester or the like. Sulfonamides are prepared from the corresponding amines by literature methods. Tetrazoles are prepared from the corresponding acid halide, e.g., the acid chloride, by literature methods.

More specific illustrations of chemistry for making these compounds is provided in the following reaction schemes. Scheme I outlines a means for making a substituted phenylalkyl tail which is R.

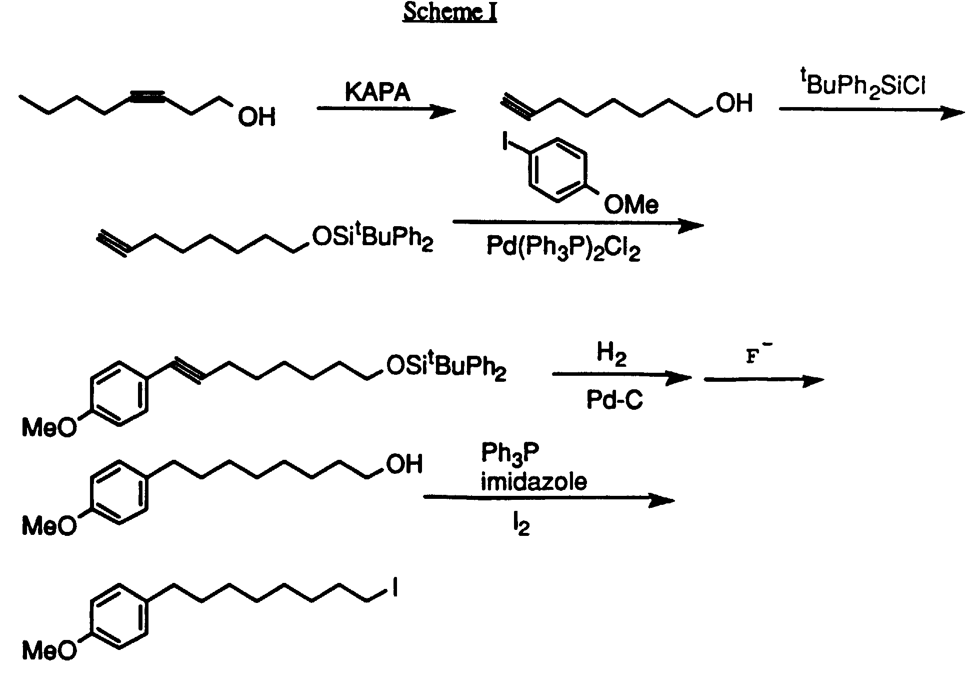

The starting alcohol, represented here as the 3-octyn-1-ol, is commercially available (Lancaster Synthesis). To migrate the triple bond to the ω-carbon, KH and 1,3-diaminopropane are combined and stirred to a homogeneous mix. This can be done at ambient temperature or thereabouts. This mix is then cooled, preferably to about 0°C or thereabouts, whereupon the alcohol is added. Stirring is then commenced at about room temperature for 15 to 20 hours or so. Water is added to quench the reaction and the product is recovered.

Protecting the alcohol is accomplished by forming a silyl ether illustrated here as the *t*-butyldiphenylsilyl ether. Other silyl ethers could be used. The alcohol is dissolved in a polar solvent, for example dimethylformamide, and imidazole is added followed by the desired silane. All this is carried out under an inert atmosphere such as argon. Ambient temperature is acceptable for effecting the reaction.

Adding the phenyl group is done in a dry environment using an amine for a solvent and an inert atmosphere. To a flask containing a solvent such as triethylamine under argon is added the silylether followed by a halophenyl compound, eg. iodoanisole, a palladium catalyst (Ph₃P)₂PdCl₂ and CuI, both of the latter in catalytic amounts. Heat is used to effect the reaction, usually a temperature of up to about 50°C will be sufficient. Two or more hours, up to six but often about four at the elevated temperature will usually cause the reaction to go to completion.

The triple bond is then saturated, preferably by catalytic hydrogenation. For example, the silyl ether can be dissolved in a saturated solvent such as an alcohol, a heavy metal catalyst added (Pd-C) and the mixture put under H₂ for a time sufficient to reduce the triple bond. Stirring for 2 to 6 hours will usually effect the reaction.

Recovering the alcohol is done by treating the silyl ether with a fluoride source such as tetrabutylammonium fluoride. Reactants are combined at a mildly reduced temperature, eg. 0°C, then the reaction is allowed to run its course at ambient temperature or there about. Several hours may be needed for the reaction to go to completion. Product was recovered by extraction means.

Converting the alcohol to the iodo compound is accomplished using a phosphine, imidazole and I₂. In actual practice, this transformation is accomplished by adding to a solution of alcohol under argon, a molar excess of triphenylphosphine, for example, and a three-fold excess of imidazole followed by iodine. Materials are combined at room temperature, but then the reaction pot may be heated to between 50 - 70°C for a brief period, 10 minutes to an hour to complete the reaction. Standard procedures are then used to recover and purify the product.

Scheme II illustrates an alternative process for making R groups.

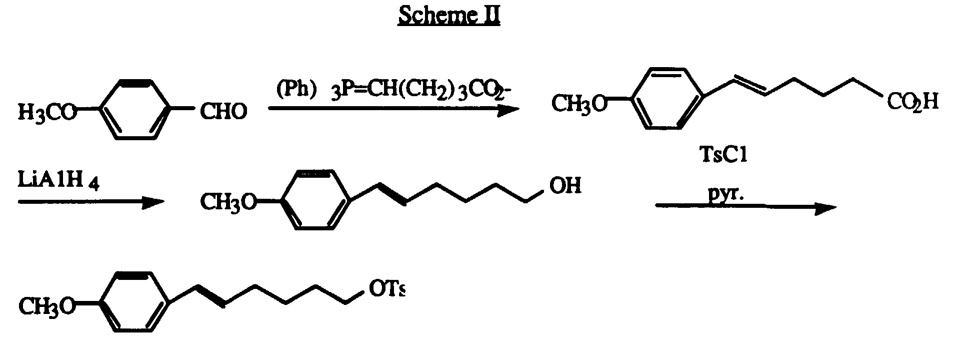

While the methoxyphenyl compound is illustrated here, this series of steps and reagents may be used to make other substituted-w-phenylaliphatic groups denoted by R. The starting material, the benzaldehydes, are commercially available or can be readily made by known methods.

To make the acid, first an alkylsilazide is added to an inert solvent under an inert atmosphere. Then the phosphonium salt is added. This addition can be done at room temperature or thereabouts. After a brief period of mixing, this mixture is usually a suspension, the benzaldehyde is added slowly at about room temperature. A slight molar excess of the phosphonium salt is employed. After an additional brief period of stirring at about room temperature, the reaction is quenched with water. The solution is acidified and the acid extracted with a suitable organic solvent. Further separatory and purification procedures may be employed as desired.

The alcohol is made by reducing the acid using a reducing agent. Lithium aluminum hydride or similar reducing agents may be employed, and conditions may be varied as needed to effect the reduction.

The tosylate is prepared in an inert solvent employing a base such as pyridine. Suitable conditions include carrying out the reaction at room temperature or thereabouts for a period of 1 to 5 hours. Other leaving groups similar in function to the tosylate may be prepared and will be useful as a means for forming the R moiety.

These procedures can be used to make the full spectrum of radicals represented by R where it has a terminal phenyl group, including the substituted phenylaliphatic radicals.

Benzyl mercaptans, or analogous compounds where m is 1 or greater, are commercially available or may be made by the process of Scheme III.

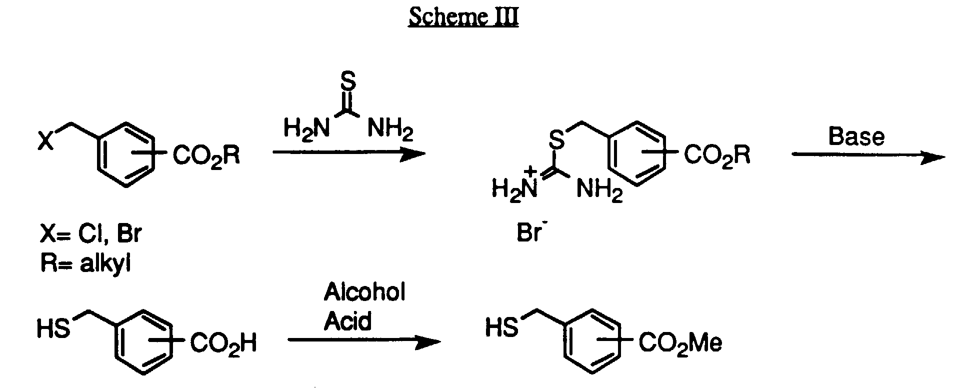

Starting material, the haloalkylbenzoates, are commercially available or can be made by methods known in the art. Thiourea is added to a solution of haloalkylbenzoate at ambient temperature or thereabouts. Any appropriate solvent may be used, acetone for example. A precipitate of the thiouronium salts should form under these conditions. The precipitate is collected and dissolved in water and the pH adjusted to about 10.5 with a base, for example a solution of NaOH. Refluxing is then commenced for between 1 and 4 hours. Product, as the free acid, is then recovered by some other separatory and purification means. Esterification is then carried out by mixing the acid with an alcohol, bubbling HCl through the solution, and letting sit the resulting solution for a time not more than several days; two days usually is sufficient to effect the reaction.

Compounds of formula I where Z is oxygen can be made by the sequence of steps given in Scheme IV.

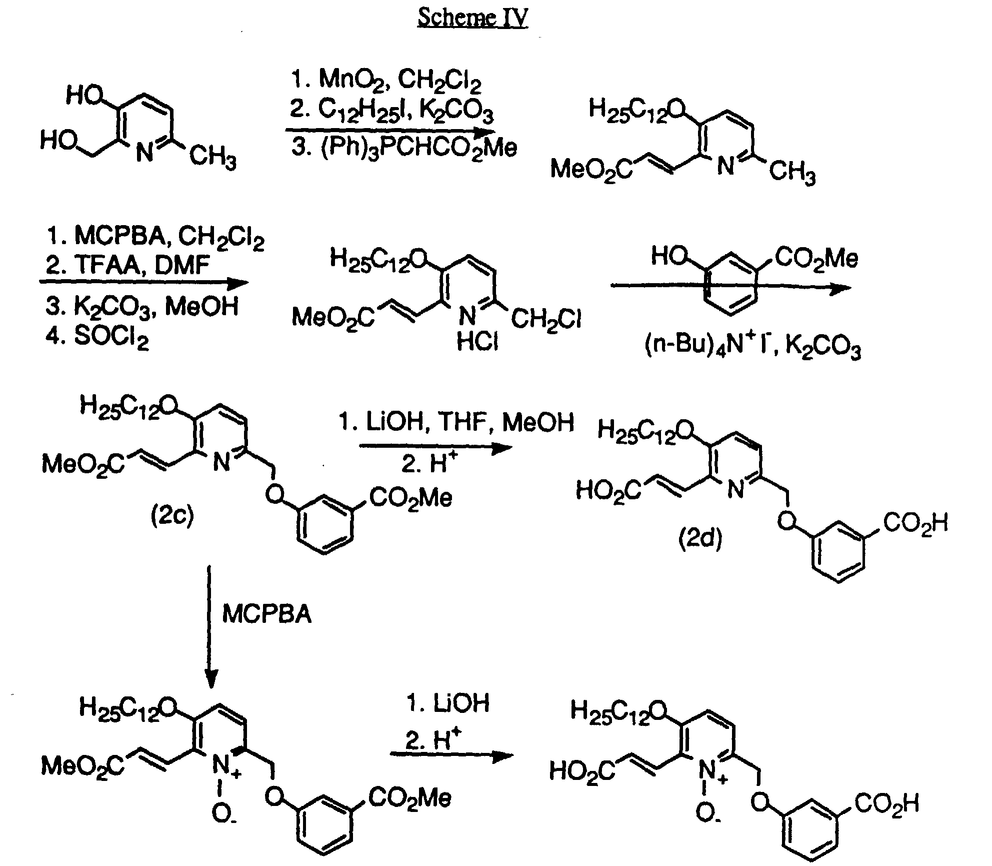

The starting material is available from Aldrich. It is treated with a mild oxidizing agent such as MnO₂ to oxidize the 2-hydroxyethyl group to the corresponding aldehyde. The R group is then formed. In this case an ether is prepared under basic conditions using an a-halo intermediate. A tosylate made as per Scheme III, can also be used in this step. Introducing the acid function at position 2 is accomplished by means of a triphenylphosphoranylidene reagent. The acetate form is illustrated here but other similar reagents could be used. The N-oxide is then formed by means of a peroxy acid. Trifluoroacetic anhydride is used to oxidize the 6-position methyl group. This hydroxymethyl group is then convened to the corresponding halide, (in the hydrohalide form) in this case the chloride, by means of thionyl chloride. An alkyl hydroxybenzoate is then reacted with the 6-chloromethyl compound in the presence of tetrabutylammonium iodide and a weak base. The resulting diester can be hydrolyzed to the salt or, further, acidified to give the free acid. An oxidant can be used to regenerate the N-oxide which can then be treated with base to hydrolyze the esters. Esters can be convened to salts, the free acids and other derivatives. Catalytic hydrogenation can be used to reduce the double bond in the R₁ group described here.

Scheme V illustrates a method for making compounds where Z is a S and m is 0.

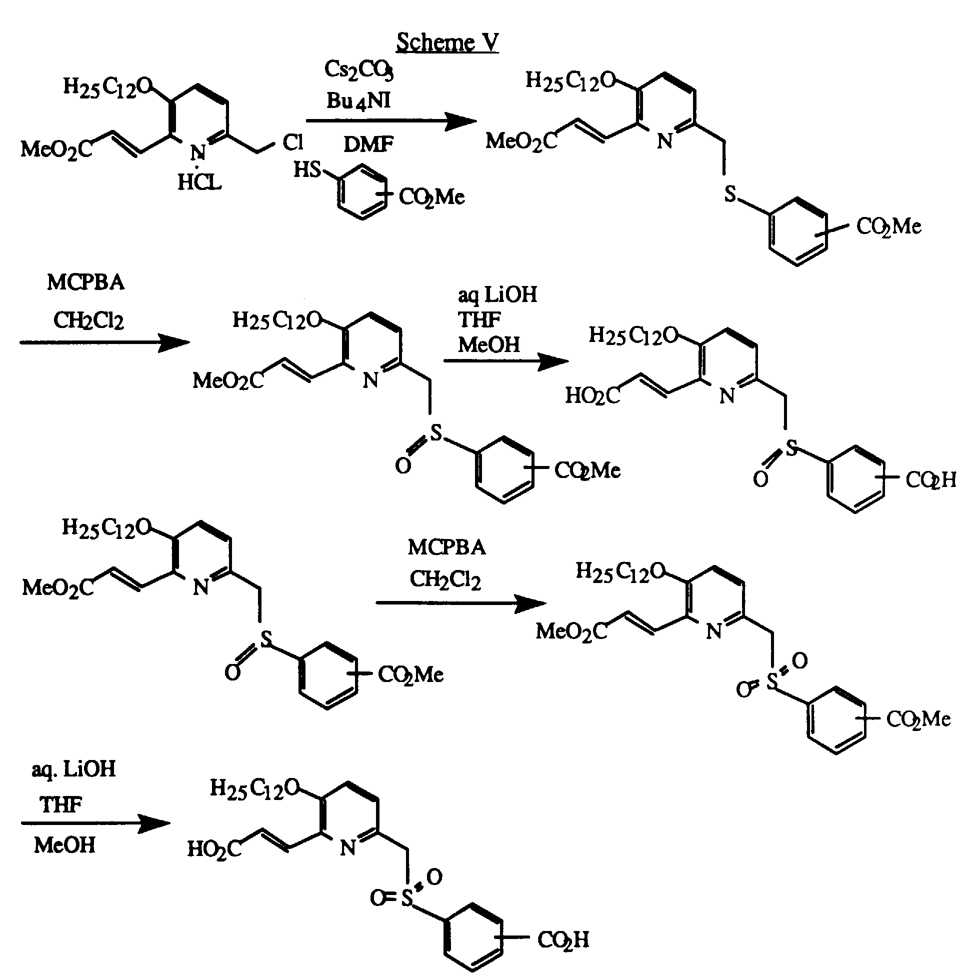

The starting hydrochloride is described in Scheme IV. Instead of treating the hydrochloride with an alcohol, in this instance the mercapto analog of the hydroxybenzoate described above is used. The resulting thioether can be hydrolyzed to give the salt or treated further to give the free acid from which other derivatives of the carboxyl function can be prepared, including alcohols and aldehydes. Also, the double bond in the R₁ group can be reduced by catalytic means using a heavy metal catalyst and hydrogen.

Once the thioether is prepared, the sulfone and sulfoxide can be prepared by treating the thioether with an oxidizing agent. A peroxy acid or other oxidizing agent can be used.

A method for making compounds where R is alkyl or subsituted alkyl is given in Scheme VI

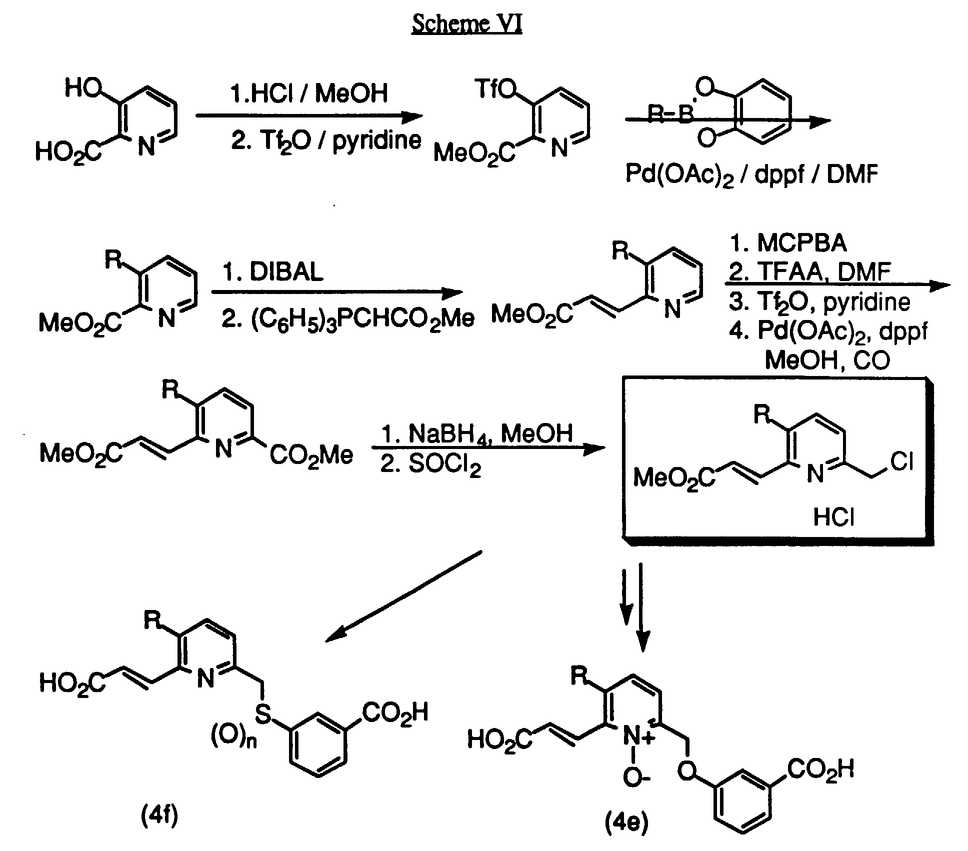

In this Scheme, 2-hydroxypicolinic acid is converted to the alkyl ester using the corresponding alcohol and an acid to catalyze the reaction. The hydroxyl group is then converted to the trifluoromethysulfonate by means of trifluoromethanesulfonic anhydride and a base, e.g. pyridine. The lipid tail is attached using the appropriate alkyl catechol boronate under palladium coupling conditions. For example, 1-iododecene and catechol borane are reacted to form the alkyl catechol boronate. Then the alkylation reaction is effected using Pd(OAc)₂. The ester is reduced to the corresponding aldehyde with a hydride such as diisobutylaluminum hydride (DIBAL). A Wittig olefination is then carried out using, for example, methyl(triphenylphosphoranylidene)acetate. The resulting pyridyl methyl acrylate is then oxidized to the N-oxide with an oxidizing agent such as 3-chloroperoxybenzoic acid. This oxide is then rearranged to the 2-pyridone with trifluoroacetic anhydride. A trifluoromethylsulfonate is then formed using trifluoromethanesulfonic anhydride and pyridine. Carbomethylation is then effected by means of Pd(OAc)₂, a simple alcohol, and carbon monoxide. Selectively reducing the pyridyl-ester (using a hydride such as NaBH₄ in a low molecular weight alcohol) yields the 2-(hydroxymethyl)-pyridine. This compound is treated with thionyl chloride to form the 6-chloromethyl compound. This intermediate is transformed to the ethers or thioether of formula I in the same manner as is illustrated in Schemes IV - VI.

Pharmaceutical compositions of the present invention comprise a pharmaceutical carrier or diluent and some amount of a compound of the formula (I). The compound may be present in an amount to effect a physiological response, or it may be present in a lesser amount such that the user will need to take two or more units of the compositon to effect the treatment intended. These compositions may be made up as a solid, liquid or in a gaseous form. Or one of these three forms may be transformed to another at the time of being administered such as when a solid is delivered by aerosol means, or when a liquid is delivered as a spray or aerosol.

The nature of the composition and the pharmaceutical carrier or diluent will, of course, depend upon the intended route of administration, for example parenterally, topically, orally or by inhalation.

For parenteral administration the pharmaceutical composition will be in the form of a sterile injectable liquid such as an ampule or an aqueous or non-aqueous liquid suspension.

For topical administration the pharmaceutical composition will be in the form of a cream, ointment, liniment, lotion, pastes, and drops suitable for administration to the eye, ear, or nose.

For oral administration the pharmaceutical composition will be in the form of a tablet, capsule, powder, pellet, atroche, lozenge, syrup, liquid, or emulsion.

When the pharmaceutical composition is employed in the form of a solution or suspension, examples of appropriate pharmaceutical carriers or diluents include: for aqueous systems, water; for non-aqueous systems, ethanol, glycerin, propylene glycol, corn oil, cottonseed oil, peanut oil, sesame oil, liquid parafms and mixtures thereof with water; for solid systems, lactose, kaolin and mannitol; and for aerosol systems, dichlorodifluoromethane, chlorotrifluoroethane and compressed carbon dioxide. Also, in addition to the pharmaceutical carrier or diluent, the instant compositions may include other ingredients such as stabilizers, antioxidants, preservatives, lubricants, suspending agents, viscosity modifiers and the like, provided that the additional ingredients do not have a detrimental effect on the therapeutic action of the instant compositions.

The pharmaceutical preparations thus described are made following the conventional techniques of the pharmaceutical chemist as appropriate to the desired end product.

Formulations for treating atopic or contact dermatitis can take the form of oral or topical preparations. Topically applied formulations are preferred. Ointments, creams, liniments, lotions, pastes and similar preparations are examples of preferred topical formulations. Aerosols may also be used. These dosage forms will contain between 0.01 and 5 percent by weight of the active ingredient.

Usually a compound of formula I is administered, that is applied, to a subject in a composition comprising a nontoxic amount sufficient to produce an inhibition of the symptoms of a disease state. When administered orally, the dosage of the composition is selected from the range of from 50 mg to 1000 mg of active ingredient for each administration. For convenience, equal doses will be administered 1 to 5 times daily with the daily dosage regimen being selected from about 50 mg to about 5000 mg. When a topical formulation is used, the amount applied will depend on the size of the affected area and the severity and progress of the disease, i.e., atopic dermatitis or contact dermatitis.

Atopic dermatitis is a chronic eruption occurring in adolescents and adults. It's origin is not known although allergic, hereditary and psychogenic factor appears to be involved. The lesions occur chiefly on the flural surfaces of the knees and elbows, but may involve other areas. They are marked by lichenfication, excoriations, and crusting. In infants and young children the condition is sometimes called *infantile eczema* or *Besnier's prurigo. It is* also called *allergic dermatitis, flexural eczema,* and *disseminated neurodermatitis.*

Contact dermatitis is an acute allergic inflammation of the skin caused by contact with various substances of a chemical, animal, or vegetable nature to which delayed hypersensitivity has been acquired; when severe, it is called *dermatitis venata.*

Treatment may be carried out in dosage units at suitable intervals or in single doses as needed. Usually this method will be practiced when relief of symptoms is specifically required. However, the method is also usefully carried out as continuous or prophylactic treatment. It is within the skill of the art to determine by routine experimentation the effective dosage to be administered from the dose range set forth above, taking into consideration such factors as the degree of severity of the condition or disease being treated, and so forth.

No toxicity is expected to be encountered when the method of this invention is carried out in conformity with the instructions set forth herein and good medical practice.

### Specific Embodiments

The following examples are given to illustrate how to make and use the compounds of this invention.

The compounds of this invention can be made by the processes set out in copending U.S. applications USSN 08/211063 filed 18 July 1994 (arising from PCT application PCT//US92/07466 published as WO 93/06085) and USSN 08/356358 filed 19 December 1994 (arising from PCT/US93/06234 published as WO 94/00437).

### Example 1

### Topical formulations

Formulations for pharmaceutical use incorporating compounds of the present invention can be prepared in various forms and with numerous excipients. Means for making various formulations can be found in standard texts such as Remington's Pharmaceutical Sciences, and similar publications and compendia. Specific examples of formulations are given below.

| Ointments | |
|---|---|
| Hydrophyllic Petrolatum | |
| Ingredients | Amount (% Weight/weight) |
| Cholesterol | 30.0g |
| Stearyl Alcohol | 30.0g |
| White Wax | 78.0g |
| Active Ingredient | 2.0g |
| White Petrolatum | 860.0g |

The stearyl alcohol, white wax and white petrolatum are melted together (steam bath for example) and cholesterol and the active ingredient are added. Stirring is commenced and continued until the solids disappear. The source of heat is removed and the mix allowed to congeal and packaged in metal or plastic tubes.

| Emulsion Ointment | |
|---|---|
| Ingredients | Amount (% W/W) |
| Methylparaben | 0.25g |
| Propylparaben | 0.15 |
| Sodium Lauryl Sulfate | 10.0g |
| Active Ingredient | 5.0g |
| Propylene Glycol | 120.0g |
| Stearyl Alcohol | 250.0g |
| White Petrolatum | 250.0g |
| Purified Water | QS to 1000.0g |

The stearyl alcohol and white petrolatum are combined over heat. Other ingredients are dissolved in water, then this solution is added to the warm (ca 50 to 100°C) alcohol/petrolatum mixture and stirred until the mixture congeals. It can then be packed in tubes or another appropriate package form.

## Claims

1. The use of at least one compound of formula I: 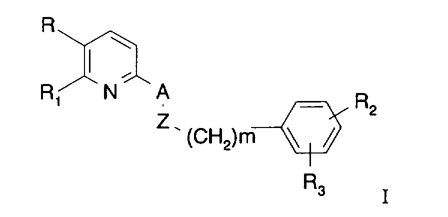 or an N-oxide, or a pharmaceutically acceptable salt thereof,
where
A is CH₂ and Z is S(O)_{q} where q is 0, 1 or 2; CHOH; C=O; NRₓ; or O; or
A is C=O and Z is NRₓ;
m is 0 - 5;
R is C₁ to C₂₀-aliphatic, unsubstituted or substituted phenyl C₁ to C₁₀-aliphatic where substituted phenyl has one or more radicals selected from the group consisting of lower alkoxy, lower alkyl, trihalomethyl, and halo, or R is C₁ to C₂₀-aliphatic-O-, or R is unsubstituted or substituted phenyl C₁ to C₁₀-aliphatic-O-where substituted phenyl has one or more radicals selected from the group consisting of lower alkoxy, lower alkyl, trihalomethyl, and halo;
R₁ is -(C₁ to C₅ aliphatic)R₄, -(C₁ to C₅ aliphatic)CHO, -(C₁ to C₅ aliphatic)CH₂OR_{8,} -R₄, -CH₂OH, or CHO;
R₂ is H, halo, lower alkyl, lower alkoxy, -CN, -(CH₂)ₙR₄, -CH(NH₂)(R₄), or -(CH₂)ₙR₉ where n is 0 - 5 and where R₉ is -N(R₇)₂ where each R₇ is independently H, or an aliphatic group of 1to 10 carbon atoms, or acyl of 1-6 carbon atoms, or a cycloalkyl-(CH₂)ₙ- group of 4 to 10 carbons where n is 0-3, or both R₇ groups form a ring which includes the nitrogen and having 4 to 6 carbons;
R₃ is hydrogen, lower alkyl, lower alkoxy, halo, -CN, R₄, NHCONH₂, or OH;
each R₄ group is independently -COR₅ where R₅ is -OH, a pharmaceutically acceptable ester-forming group -OR₆, or -OX where X is a pharmaceutically acceptable cation, or R₅ is -N(R₇)₂ where each R₇ is independently H, or an aliphatic group of 1 to 10 carbon atoms, or a cycloalkyl-(CH₂)ₙ- group of 4 to 10 carbons where n is 0-3, or both R₇ groups form a ring having 4 to 6 carbons, or R₄ is a sulfonamide, or an amide, or tetrazol-5-yl; and
R₈ is hydrogen, C₁ to C₆ alkyl, or C₁ to C₆-acyl;
in the manufacture of a medicament for the treatment of atopic dermatitis or contact dermatitis in a mammal.

2. The use according to claim 1 where, in formula I, Z is S(O)_{q} and m is 0 or 1.

3. The use according to claim 1 or 2 where, in formula I, R is alkoxy of 8 to 15 carbon atoms or unsubstituted or substituted phenyl-C₁ to C₁₀-alkyl-O-where substituted phenyl is substituted with fluoro, trifluoromethyl or methoxy and R₁ is R₄CH=CH- or R₄CH₂CH₂-_{.}

4. The use according to claim 2 where, in formula I, q is 0 and m is 0.

5. The use according to claim 4 wherein the compound of formula I is 3-[1-thia-2-[2-(E-2-carboxyethenyl)-3-[8-(4-methoxyphenyl)octyloxy]-6-pyridyl]ethyl]benzoic acid, or a pharmaceutically acceptable salt thereof.

6. The use according to any one of claims 1 to 4 where, in formula I, R is unsubstituted or substituted phenyl-C₂ to C₁₀ alkoxy.

7. The use according to claim 2 wherein the compound of formula I is (E)-3-[3-[2-phenethyloxy]-6-[(2,6-dichlorophenylsulfinyl)methyl]-2-pyridinyl]-2-propenoic acid, or a pharmaceutically acceptable salt thereof.

## Patentansprüche

1. Verwendung mindestens einer Verbindung der Formel I: 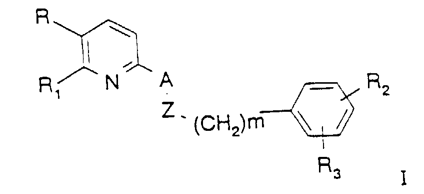 oder eines N-Oxides oder eines pharmazeutisch verträglichen Salzes davon, wobei
A CH₂ und Z S(O)_{q}, wobei q 0, 1 oder 2 ist; CHOH; C=O; NRₓ oder 0 bedeutet; oder
A C=O und Z NRₓ bedeutet;
m 0-5 ist;
R einen C₁ bis C₂₀- aliphatischen Rest, einen C₁ bis C₂₀-aliphatischen Rest mit einem unsubstituierten oder substituierten Phenylrest darstellt, wobei der substituierte Phenylrest einen oder mehrere Reste aufweist, die aus Niederalkoxy-, Niederalkyl-, Trihalogenmethylresten und Halogenatomen ausgewählt sind, oder R einen C₁ bis C₂₀-aliphatischen Oxyrest darstellt, oder R einen C₁ bis C₂₀-aliphatischen Oxyrest mit einem unsubstituierten oder substituierten Phenylrest darstellt, wobei der substituierte Phenylrest einen oder mehrere Reste aufweist, die aus Niederalkoxy-, Niederalkyl-, Trihalogenmethylresten und Halogenatomen ausgewählt sind;
R₁ einen Rest -(C₁ bis C₅-aliphatisch)R₄, -(C₁ bis C₅-aliphatisch)CHO, -(C₁ bis C₅-aliphatisch)CH₂OR₈, -R₄, -CH₂OH oder CHO darstellt;
R₂ H, ein Halogenatom, einen Niederalkylrest, Niederalkoxyrest, -CN, -(CH₂)ₙR₄, -CH(NH₂)(R₄) oder -(CH₂)ₙR₉ darstellt, wobei n 0 - 5 ist und wobei R₉ einen Rest N(R₇)₂ bedeutet, in dem jeder Rest R₇ unabhängig voneinander ein Wasserstoffatom, einen aliphatischen Rest von 1 bis 10 Kohlenstoffatomen, einen Acylrest von 1 - 6 Kohlenstoffatomen oder einen Cycloalkyl-(CH₂)ₙ-Rest mit 4 bis 10 Kohlenstoffatomen, wobei n 0 - 3 ist, darstellt, oder beide Reste R₇ einen Ring bilden, der das Stickstoffatom einschließt und 4 bis 6 Kohlenstoffatome hat;
R₃ ein Wasserstoffatom, einen Niederalkylrest, Niederalkoxyrest, Halogenatom, CN, R₄, NHCONH₂ oder OH darstellt;
jeder Rest R₄ unabhängig voneinander einen Rest -COR₅ darstellt, wobei R₅ -OH, eine pharmazeutisch verträgliche Ester bildende Gruppe -OR₆ oder -OX bedeutet, in dem X ein pharmazeutisch verträgliches Kation ist, oder R₅ einen Rest N(R₇)₂ bedeutet, in dem jeder Rest R₇ unabhängig voneinander ein Wasserstoffatom, einen aliphatischen Rest von 1 bis 10 Kohlenstoffatomen oder einen Cycloalkyl-(CH₂)ₙ-Rest mit 4 bis 10 Kohlenstoffatomen, wobei n 0 - 3 ist, darstellt, oder beide Reste R₇ einen Ring mir 4 bis 6 Kohlenstoffatomen bilden, oder R₄ ein Sulfonamid oder ein Amid oder eine Tetrazol-5-yl-Gruppe darstellt; und
R₈ ein Wasserstoffatom, einen C₁ bis C₆-Alkylrest oder einen C₁ bis C₆-Acylrest darstellt; zur Herstellung eines Arzneimittels für die Behandlung von atopischer Dermatitis oder Kontaktdermatitis in einem Säuger.

2. Verwendung nach Anspruch 1, wobei in der Formel I Z S(O)_{q} bedeutet und m 0 oder 1 ist.

3. Verwendung nach einem der Ansprüche 1 oder 2, wobei in der Formel I R einen Alkoxyrest mit 8 bis 15 Kohlenstoffatomen oder einen C₁ bis C₁₀-alkyloxyrest mit einem unsubstituierten oder substituierten Phenylrest darstellt, wobei der substituierte Phenylrest mit Fluor, Trifluormethyl oder Methoxy substituiert ist, und R₁ einen Rest R₄CH=CH- oder R₄CH₂CH₂- darstellt.

4. Verwendung nach Anspruch 2, wobei in Formel I q 0 und m 0 ist.

5. Verwendung nach Anspruch 4, wobei es sich bei der Verbindung der Formel I um 3-[1-Thia-2-[2-(E-2-carboxyethenyl)-3-[8-(4-methoxyphenyl)octyloxy]-6-pyridyl]ethyl]benzoesäure oder ein pharmazeutisch verträgliches Salz davon handelt.

6. Verwendung nach einem der Ansprüche 1 bis 4, wobei in der Formel I R einen unsubstituierten oder substituierten Phenyl-C₁ bis C₁₀-Alkoxyrest darstellt.

7. Verwendung nach Anspruch 2, wobei es sich bei der Verbindung der Formel I um (E)-3-[3-[2-phenethyloxy]-6-[(2,6-dichlorphenylsulfinyl)methyl]-2-pyridinyl]-2-propensäure oder ein pharmazeutisch verträgliches Salz davon handelt.

## Revendications

1. Utilisation d'au moins un composé de formule (I) : 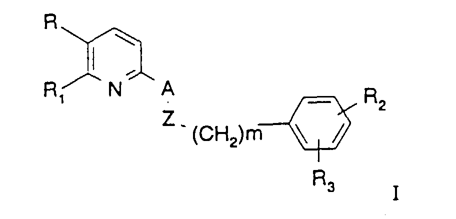 ou d'un de ses N-oxydes, ou bien d'un de ses sels pharmaceutiquement acceptables,
formule dans laquelle
. A représente un groupe CH₂ et Z représente un groupe S(O)_{q} dans lequel q est égal à 0, 1 ou 2 ; CHOH ; C=O ; NRₓ ; ou O ; ou bien
A représente un groupe C=O et Z représente un groupe NRₓ ;
m a une valeur de 0 à 5 ;
R représente un groupe aliphatique en C₁ à C₂₀, (phényle non substitué ou substitué)-(aliphatique en C₁ à C₁₀) dans lequel le groupe phényle substitué comporte un ou plusieurs radicaux choisis dans le groupe consistant en des radicaux alkoxy inférieur, alkyle inférieur, trihalogénométhyle et halogéno, ou R représente un groupe (aliphatique en C₁ à C₂₀)-O-, ou bien R représente un groupe (phényle non substitué ou substitué)-(aliphatique en C₁ à C₁₀)-O- dont le groupe phényle substitué porte un ou plusieurs radicaux choisis dans le groupe consistant en des radicaux alkoxy inférieur, alkyle inférieur, trihalogénométhyle ou halogéno ;
R₁ représente un groupe (aliphatique en C₁ à C₅)R₄, - (aliphatique en C₁ à C₅)CHO, -(aliphatique en C₁ à C₅)CH₂OR₈, -R₄, -CH₂OH ou CHO ;
R₂ représente H, un groupe halogéno, alkyle inférieur, alkoxy inférieur, -CN, -(CH₂)ₙR₄, -CH(NH₂)(R₄) ou -(CH₂)ₙR₉ dans lequel n a une valeur de 0 à 5 et R₉ représente un groupe -N(R₇)₂ dans lequel chaque groupe R₇ représente, indépendamment, H ou un groupe aliphatique ayant 1 à 10 atomes de carbone, ou un groupe acyle ayant 1 à 6 atomes de carbone ou bien un groupe cycloalkyl-(CH₂)ₙ ayant 4 à 10 atomes de carbone dans lequel n a une valeur de 0 à 3, ou bien les deux groupes R₇ forment un noyau qui comprend l'atome d'azote et qui a 4 à 6 atomes de carbone ;
R₃ représente l'hydrogène, un groupe alkyle inférieur, alkoxy inférieur, halogéno, -CN, R₄, NHCONH₂ ou OH ;
chaque groupe R₄ représente, indépendamment, un groupe -COR₅ dans lequel R₅ représente un groupe -OH, un groupe -OR₆ formant un ester pharmaceutiquement acceptable, ou un groupe -OX dans lequel X représente un cation pharmaceutiquement acceptable, ou bien R₅ représente un groupe -N(R₇)₂ dans lequel chaque groupe R₇ représente, indépendamment, H, ou un groupe aliphatique ayant 1 à 10 atomes de carbone, ou un groupe cycloalkyl-(CH₂)ₙ- de 4 à 10 atomes de carbone dans lequel n a une valeur de 0 à 3, ou bien les deux groupes R₇ forment un groupe ayant 4 à 6 atomes de carbone, ou bien R₄ représente un groupe sulfonamide, amide ou tétrazole-5-yle ; et
R₈ représente l'hydrogène, un groupe alkyle en C₁ à C₆ ou acyle en C₁ à C₆ ;
dans la production d'un médicament destiné au traitement de la dermatite atopique ou de la dermatite de contact chez un mammifère.

2. Utilisation suivant la revendication 1, dans laquelle, dans la formule I, Z représente un groupe S(O)_{q} et m est égal à 0 ou 1.

3. Utilisation suivant la revendication 1 ou 2, dans laquelle, dans la formule I, R représente un groupe alkoxy ayant 8 à 15 atomes de carbone ou un groupe (phényle non substitué ou substitué)-(alkyle en C₁ à C₁₀)-O- dans lequel le groupe phényle substitué est substitué avec un substituant fluoro, trifluorométhyle ou méthoxy et R₁ représente un groupe R₄CH=CH- ou R₄CH₂CH₂-.

4. Utilisation suivant la revendication 2, dans laquelle, dans la formule I, q est égal à 0 et m est égal à 0.

5. Utilisation suivant la revendication 4, dans laquelle le composé de formule I est l'acide 3-[1-thia-2-[2-(E-2-carboxyéthényl) -3-[8-(4-méthoxyphényl)octyloxy]-6-pyridyl]éthyl]benzoïque ou un de ses sels pharmaceutiquement acceptables.

6. Utilisation suivant l'une quelconque des revendications 1 à 4, dans laquelle, dans la formule I, R représente un groupe (phényle non substitué ou substitué)(alkoxy en C₂ à C₁₀).

7. Utilisation suivant la revendication 2, dans laquelle le composé de formule I est l'acide (E)-3-[3-[2-phénéthyloxy]-6-[(2,6-dichlorophénylsulfinyl)méthyl]-2-pyridinyl]-2-propénoïque ou un de ses sels pharmaceutiquement acceptables.
